Europäisches Patentamt

(19) European Patent Office

Office européen des brevets

(11) Publication number: **0 434 445 A2**

# EUROPEAN PATENT APPLICATION

(21) Application number: 90314074.7

(22) Date of filing: 20.12.90

(51) Int. Cl.$^5$: **C07F 1/00, A61K 31/30**

(30) Priority: 20.12.89 MX 18801

(43) Date of publication of application:
26.06.91 Bulletin 91/26

(84) Designated Contracting States:
**CH DE FR GB IT LI**

(71) Applicant: **UNIVERSIDAD NACIONAL AUTONOMA DE MEXICO**
90 Piso de la Torre de Rectoria, Ciudad Universitaria
Mexico 04510 D.F. (MX)

(72) Inventor: **Azuara, Lena Ruiz Dra.**
**Gregorio Lopez Y Fuentes 332 Villa de Cortes**
**C.P. 03530 Mexico D.F. (MX)**

(74) Representative: **Drever, Ronald Fergus et al**
**Swindell & Pearson 48, Friar Gate**
**Derby DE1 1GY (GB)**

(54) **Process to obtain new mixed copper aminoacidate complexes from phenanthrolines and their alkyl derivatives to be used as anticancerigenic agents.**

(57) This invention refers to a process to obtain new mixed copper aminoacidates from phenanthrolines and their alkyl derivates of an aromatic type to be used as anticancerigenic, agents preferably with a therapeutic use for the treatment of liquid and solid cancerigenic tumour such as leukemia. The complexes obtained are of the [Cu (N-N) (N-O)]$^{+-}$ NO$_3$ type in which the N-N ligand corresponds to 4, 7-dimethyl-1, 10 phenanthroline and the N-O ligand preferably correponds to one of the aminoacidates such as tyrosine-alaninate, treaninate, triptophanate, valinate, isoleucinate, cisteinate, diglycinate, phenylalaninate, glycinate, histidinate, serinate, tyrosinate, aspartate, alaninate and phenylalaninate. The process is characterized because it includes the following steps: making an aqueous solution based on an aliphatic alcohol and 4, 7-dimethyl-1, 10 phenanthroline react with a copper complex preferably Cu NO$_3$ 5H$_2$O at room temperature, and immediately after making the obtained product react in an aqueous aminoacidate solution adjusting a slightly alkaline pH.

EP 0 434 445 A2

# PROCESS TO OBTAIN NEW MIXED COPPER AMINOACIDATE COMPLEXES FROM PHENANTHROLINES AND THEIR ALKYL DERIVATES TO BE USED AS ANTICANCERIGENIC AGENTS

## Background of the Invention

A type of drug for which a correlation between biological activity and structure has been found is the metal chelating agent such as iron, ruthenium, cobalt, manganese, zinc and copper (Dwyer, F.P. et al. Nature. 179, (1956), 425-426), Chelating agents can be designed to inactivate bacteria, viruses, and fungi by capturing the metallic ions necessary for the metebolism of these microorganisms. They can also be supplied with metallic ions that prove toxic to them.

Studies related to the composition of physiological fluids have shown that the metallic ions present in these systems are largely found in the form of mixed complexes. Here the term mixed complex must be understood to mean all those coordination complexes with two or three chelate type ligands that are different one from the other, excluding the solvent of the chelate ligant category.

Sigel and colleagues (Sigel, H. et. al., JACS 99 : 13 (1976), 4489-4496) have shown that in many cases the enzymatic action depends on an elementary process in which an enzyme-substrate complex is generated that presents characteristics analogous to those of a mixed complex.

Certain metallic chelates supplied in small concentrations are active against some bacteria, fungi, viruses and some tumourous cells.

It is well known, for instance, that **Staphylococcous pyrogenes**, which has been detected in infected wounds and has proved to be highly resistent to the action of many standard antibiotics, perishes in the present of the iron (tetramethyl-1, 1, 10 phenanthroline)$_3$ (acetylacetonate)$^+$ and rethenium (tetramethyl-1, 10-phenanthroline)$_2$ (acethylacetonate)$^+$complexes. The biological activity of these satured chelate complexes is principally due to the stereochemistry of the complex as a whole, the ionic nature of the coordination sphere, the nature of the coordination center, the lypophilicity of the ligands, the redox potentials of the complexes, and the thermodynamic stability and kinetic of the metallic chelates.

Volpir and colleagues have studied the catalytic activity of the Co (II) an Cu (II) complexes with orthophenanthrolines in the oxidation processes of the NADH, $Q_4$ $H_2$ (quinone), $Q_9$ $H_2$ (ubiquinone) and cytochrome C susbtrates.

The [Cu (2, 9-dimethyl-1, 10-phenanthroline)]$^+$ C1$_2$ complex is an effective inhibitor of the growth of the plasmodium **Micoplasmacillisepticum**, a very dangerous pathogenetic micro-organism that induces pulmonary diseases in man. D. R. Williams has calculated the distribution percentages of the Cu (II), Fe (II), Mn (II) and Zn (II) ions with low weight ligands, the predominant molecules in human blood plasma ; Cu (hys) (Cys) ; (ulhys)$_2$ and Cu (hys) (CysH)$^+$ are among the most abundant complexes.

Mention is made in the literature of studies on the absorption of the Mn (gly)$_2$ and Mn (L-ala)$_2$ chelate complexes through the intestinal wall in dogs.

Kwik and colleagues have reported the synthesis of mixed complexes with the general formula [Cu (phenanthroline) (aminoacidate)] nH$_2$O, [Cu (bipyridine) (aminoacidate)]n H$_2$O, [Cu (phenanthroline) (aminoacidate) (C1, O$_2$, SO$_4$)] nH$_2$O where the aminoacidate is the glycinate, alaninate, valinate, tyrosinate, serinate, aspartate or glutamate ion.

Where the general synthetis method consists of preparing an aqueous solution with 5 mmol of CuSO$_4$·5H$_2$O in 20 ml of destilled water which is slowly added to an ethanolic solution with 8 mmols of o-phenanthroline in 20 ml of ethanol, a solution blue in colour is immediately formed. The mixture is magnetically shaken and at the same time a solution of 10 ml of HCl 0.1 M that containing one of the aminoacidates is added, immediately followed by a solution of ammonium hydroxide 1 M until the solution becomes clear. The mixture that has been thus prepared is heated and shaken for 30 minutes ; the heating process is continued until the volume is reduced by half, the product obtained is cooled in ice until a solid is formed, it is filtered, washed in small pieces in cold water and then in ethanol and is finally vacuum dried.

The applicant has developed a process to obtain new chelates by means of the synthesis and characterization of mixed chelate complexes of ion Mn (II), Fe (II), Ni (II), Co (II, III), Cu (II) Zn (II) and Ru (II) with ligands of the N-N (phenanthrolines and bipyridines), O-O (acethylacetonate and salicilaldhehydate ions) and N-O (aminocidate ions) type. These mixed chelate complexes have some type of potential biological activity.

With the ion Cu (II) in particular 19 new mixed complexes of the [Cu (N-N) (N-O)]$^+$ NO$_3$ type have been isolated.

## Summary of the Invention

This invention refers to a process to obtain new mixed copper aminoacidate complexes from phenanthrolines and their alkyl derivates of an aromatic type with anticancerigenic properties, with the formula [Cu (N-N) (N-O)]$^+$ NO$_3$, in which the N-N ligand (diimine) corresponds to 1-10-phenanthroline and its alkyl derivates preferably group 4, 7-dimethyl-1, 10-phenanthroline and the N-O ligand is an aminoacidate that corresponds principally to the following ions : tyrosin-alaninate, treoninate, triptophanate, valinate, isoleucinate, cysteinate, diglycinate, phenylalaninate, glycinate, hystidinate, serinate, tyrosinate, aspartate, alaninate and phenylalaninate, and is characterized because it comprises the steps for preparing an aqueous solution based on a copper compound, preferably $Cu (NO_3)_2 \cdot 5H_2O$ at a ratio of 30-35% copper weight/solution ; prepare an aqueo-ethanolic solution with a diimine compound at a ratio of 50-55% dimine weight/solution ; make the aqueous solutions react in a reactor at room temperature ; stabilize the copper complex obtained (monodiimine nitrate) with a regulating agent maintaining an acid pH of between 5 and 7 ; add to the complex obtained an aqueous solution of 10-15% in weight of an aminoacidate (N-O) keeping the reaction slightly alkaline to obtain copper aminoacidate aromatic complexes with anticancerigenic properties wih the formula :

1) [Cu (1, 10-phenanthroline) (thyrosine-alaninate)]$^+$ $NO_3$

2) [Cu (1, 10-phenanthroline) (treoninate)]$^+$ $NO_3$

3) [Cu (1, 10-phenanthroline) (thriptophanate)]$^+$ $NO_3$

4) [Cu (1, 10-phenanthroline) (valinate)]$^+$ $NO_3$

5) [Cu (1, 10-phenanthroline) (isoleucinate)]$^+$ $NO_3$

6) [Cu (1, 10-phenanthroline) (cysteinate)]$^+$ $NO_3$

7) [Cu (1, 10-phenanthroline) (diglycinate)]$^+$ $NO_3$

8) [Cu (1, 10-phenanthroline) (phenylalaninate)]$^+$ $NO_3$

9) [Cu (1, 10-phenanthroline) (glycinate)]$^+$ $NO_3$

10) [Cu (1, 10-phenanthroline) (hystidinate)]$^+$ $NO_3$

11) [Cu (1, 10-phenanthroline) (serinate)]$^+$ $NO_3$

12) [Cu (1, 10-phenanthroline) (tyrosinate)]$^+$ $NO_3$

13) [Cu (1, 10-phenanthroline) (aspartate)]$^+$ $NO_3$

14) [Cu (1, 10-phenanthroline) (alaninate)]$^+$ $NO_3$

15) [Cu (4, 7-dimethyl-1, 10-phenanthroline) (phenylalaninate)]$^+$ $NO_3$.

16) [Cu (4, 7 dimethyl-1, 10-phenanthroline) (glycinate)]$^+$ $NO_3$

17) [Cu (4, 7 dimethyl-1, 10-phenanthroline) (hystidinate)]$^+$ $NO_3$

18) [Cu (4, 7 dimethyl-1, 10-phenanthroline) (tyrosinate)]$^+$ $NO_3$

19) [Cu (4, 7-dimethyl-1, 10-phenanthroline) (alaninate)]$^+$ $NO_3$

## Detailed Description of the Invention

This invention refers to a process to obtain new mixed copper aminoacidate complexes from phenanthrolines and their alkyl derivates to be used as anticancerigenic agents in which $Cu (NO_3)_2 \cdot 5 H_2O$ is used as raw material ; this complex has not been used in conventional process ; the technique makes it possible to obtain crystals that are highly soluble in water and furthermore the reactions that occur during the process of this invention take place at room temperature thus avoiding the therapeutic properties of the aminoacidate obtained breaking down.

The process generally takes places in two stages according to the following :

1) To form the intemediary copper complex of the [Cu (N-N)]$^+$ $NO_3$ type, an aqueo-ethanolic solution is prepared with a content of 30-35% in weight of $Cu (NO_3)_2 \cdot 5H_2O$ and an aqueo-ethanolic solution of 50-55% in weight of the corresponding diimine ; both are immediately brought into contact in a reactor for approximately 5 minutes at room temperature. The resulting pH must be between 5 and 7.

2) To form the stabilized copper complex (monodiimine nitrate aminoacidate), an aqueous solution is added to the above mentioned component 10-15% in weight of the aminoacidate ion (N-O) adjusting the slightly alkaline pH with an aquoammoniacal solution.

The process is also characterized because it is passed through a millipore filter in order to eliminate raw materials and secondary products, to sterilize the solution and to obtain a pure product. It is concentrated by slight heating until the volume is reduced by half ; when the mixture cools, the expected product precipitates and is finally filtered, thus giving a yield of 60% ; to achieve greater purity it is recrystalized in ethanol/water (to a ratio of 30/70). The crystals are royal blue in colour.

The complexes formed as a result of this process are any of the following :

1) [Cu (1, 10-phenanthroline) (tyrosine-alaninate)]$^+$ $NO_3$

2) [Cu (1, 10-phenanthroline) (treoninate)]$^+$ $NO_3$

3) [Cu (1, 10-phenanthroline) (thriptophanate)]$^+$ $NO_3$

4) [Cu (1, 10-phenanthroline) (valinate)]$^+$ $NO_3$

5) [Cu (1, 10-phenanthroline) (isoleucinate)]$^+$ $NO_3$

6) [Cu (1, 10-phenanthroline) (cysteinate)]$^+$ $NO_3$

7) [Cu (1, 10-phenanthroline) (diglycinate)]$^+$ $NO_3$

8) [Cu (1, 10-phenanthroline) (phenylalaninate)]$^+$ $NO_3$

9) [Cu (1, 10-phenanthroline) (glycinate)]$^+$ $NO_3$

10) [Cu (1, 10-phenanthroline) (hystidinate)]$^+$ $NO_3$

11) [Cu (1, 10-phenanthroline) (serinate)]$^+$ $NO_3$

12) [Cu (1, 10-phenanthroline) (tyrosinate)]$^+$ $NO_3$

13) [Cu (1, 10-phenanthroline) (aspartate)]$^+$ $NO_3$

14) [Cu (1, 10-phenanthroline) (alaninate)]$^+$ $NO_3$

15) [Cu (4, 7-dimethyl-1, 10-phenanthroline) (phenylalaninate)]$^+$ $NO_3$

16) [Cu (4, 7-dimethyl-1, 10-phenanthroline) (glycinate)]$^+$ $NO_3$

17) [Cu (4, 7-dimethyl-1, 10-phenanthroline) (hystidinate)]$^+$ $NO_3$

18) [Cu (4, 7-dimethyl-1, 10-phenanthroline) (tyrosinate)]$^+$ $NO_3$

19) [Cu (4, 7-dimethyl-1, 10-phenanthroline) (alaninate)]$^+$ $NO_3$

Examples of the invention are given below in order to illustrate it better, not with the purpose of restricting its scope.

## EXAMPLE 1

To obtain a complex with the formula (Cu (1, 10-phenenthroline) (tyrosin-alaninate)$^+$ $NO_3$, a first aqueo-ethanolic solution was prepared in a container with 215.6 mg. of Cu $(NO_3)_2 \cdot 5H_2O$ ; a second aqueo-ethanolic solution was immediately prepared with 180 mg of 1, 10-phenanthroline ; the aqueous solutions were then made to react in a reactor at room temperature ; soon afterwards a regulating agent was added to stabilize the intermediary complex obtained, keeping the pH between 5 and 7, an aqueous solution with 302.4 mg of tyrosin-alanine was subsequently added to the intermediary complex obtained ; the reaction was kept at a pH of between 7 and 8 and regulated with an aqueo-ammoniacal solution for five minutes ; the product obtained was immediately passed through a millipore filter ; it was concentrated by slight heating and distilled in vacuum until the volume was reduced by half ; once cooled the mixture of the aromatic complex obtained from copper tyrosin-alaninate was precipitated ; it was then filtered and recrystalized in an ethanol/water mixture (with a ratio of 30/70).

The crystals obtained were royal blue in colour and their charaterization was the following :

COMPOUND FORMULA : [Cu (1, 10-phenanthroline) (tyrosin-alaninate)]$^+$ $NO_3$

ELEMENT ANALYSIS OBTAINED (%) : 51.82 C ; 4.30 H ; 10.12 N

CALCULATED ELEMENT ANALYSIS (%) : (51.74) C ; (4.13) H ; (10.06) N

BORH MAGNETONS ($\mu$, eff) : 1.83

CONDUCTIVITY (Ohms. mol. cm$^{-1}$) : 225 (water)

max visible (nm) : 626

IR (cm $^{-1}$) : 1609, 1380, 1107, 873, 846 and 827, 775, 721

(IR = Infrared analysis)

## EXAMPLE 2

A complex with the formula [Cu (1, 10-phenanthroline) (treoninate)]$^+$ $NO_3$ was obtained by preparating an aqueo-ethanolic solution in a container with 215.6 mg of Cu $(NO_3)_2 5H_2O$ ; and also preparing an aqueo-ethanolic solution with 180 mg of 1, 10-phenanthroline ; these aqueous solutions were then made to react in a reactor at room temperature ;

shortly afterwards a regulating agent was added to stabilize the intermediary complex obtained with the formula [Cu (1, 10-phenanthroline)]$^+$ $NO_3$, keeping the pH between 5 and 7 ; an aqueous solution with 141.6 mg of treonine was subsequently added to the intermediary complex obtained ; the reaction was kept at a pH of between 7 and 8, and regulated with an aqueo-ammoniacal solution for 5 minutes ; the product obtained was immediately passed through a millipore filter ; it was concentrated by slight heating and distilled in vacuum until the volume was reduced by half ; when the mixture had cooled, the aromatic complex based on copper treoninate was precipitated ; it was the filtered and recrystalized in an ethanol/water mixture at a ratio of 30/70). The crystals obtained were royal blue in colour and their characterization was the following :

COMPOUND FORMULA : [Cu (1, 10-phenanthroline) (treoninate)]$^+$ $NO_3$

ELEMENT ANALYSIS OBTAINED (%) : 45.7 C ; 3.9 H ; 13.4 N

CALCULATED ELEMENT ANALYSIS (%) : (45.34) C ; (3.81) H ; (13.22) N

BORH MAGNETONS ($\mu$, eff) : 1.75

CONDUCTIVITY (Ohms. mol. cm$^{-1}$) : 85 (water)

max visible (nm) : 611

IR (cm $^{-1}$) : 1523, 1380, 1160, 1055, 850, 790, 723, 646

## EXAMPLE 3

A complex with the formula [Cu (1, 10-phenanthroline) (triptophanate)]$^+$ $NO_3$ was obtained following the technique described in examples 1 and 2 to obtain the intermediary complex with the formula [Cu (1, 10 phenanthroline)]$^+$ $NO_3$ which was stabilized with a regulating agent to keep pH between 5 and 7, an aqueous solution was then added with 226.8 mg of triptophane ; the reaction was kept at a pH of between 7 and 8 and was regulated with an aqueo-ammoniacal solution for 5 minutes, the product obtained was immediately passed through a millipore filter ; it was concentrated with slight heating and distilled in vacuum until the volume was reduced by half ; when the mixture had cooled the aromatic complex based on copper triptophanate was precipitated ; it was filtered and recrystalized in ethanol/water (at a ratio of 30/70). The crystals obtained were royal blue in colour and their characterization was the following.

COMPOUND FORMULA : [Cu (1, 10-phenanthroline) (triptophanate)]$^+$ $NO_3$

ELEMENT ANALYSIS OBTAINED (%) : 51.0 C ; 3.8 H ; 13.5 N

CALCULATED ELEMENT ANALYSIS (%) : (50.28) C ; (3.76) H ; (13.76) N

BORH MAGNETONS : ($\mu$, eff) : 1.85

CONDUCTIVITY (Ohms. mol. cm$^{-1}$) : 135 (water)

max visible (nm) : 615

IR (cm$^{-1}$) : 1611, 1521,1380, 1141, 873, 841, 742 and 719

## EXAMPLE 4

A complex with the formula [Cu (1, 10-phenanthroline) (valinate)]$^+$ NO$_3$ was obtained following the technique described in examples 1 and 2 to obtain the intermediary complex with the formula [Cu (1, 10-phenanthroline)]$^+$ NO$_3$ which was stabilized with a regulating agent to keep pH at between 5 and 7 ; an aqueous solution was then added with 139.2 mg of valine ; the reaction was regulated at a pH of between 7 and 8 with an aqueo-ammoniacal solution for 5 minutes ; the product obtained was immediately passed with a millipore filter ; it was concentrated by slight heating and distilled in vacuum until the volume had reduced by half ; when the mixture cooled, the aromatic complex based on copper valinate was precipitated ; it was filtered and recrystalized in ethanol/water (ratio : 30/70).

The crystals obtained were royal blue in colour and their characterization was the following :

COMPOUND FORMULA : [Cu (1, 10-phenanthroline) (valinate)]$^+$ NO$_3$

ELEMENT ANALYSIS OBTAINED (%) : 48.6.C ; 4.5 H ; 13.4 N

CALCULATED ELEMENT ANALYSIS (%) : (48.40) C ; (4.30) H ; (13.28) N

BORH MAGNETONS ($\mu$, eff) : 1.79

CONDUCTIVITY (Ohms. mol. cm$^{-1}$) : 118 (water)

max visible (nm) : 654

IR (cm $^{-1}$) : 1655, 1522, 1380, 1195, 1050, 832, 765, 723, 652

## EXAMPLE 5

A complex with the formula [Cu (1, 10-phenanthroline) (isoleucinate)]$^+$ NO$_3$ was obtained ; the intermediary complex was prepared with the formula [Cu (1, 10-phenanthroline)]$^+$ NO$_3$ by means of the technique described in examples 1 and 2 and its pH was regulated at between 5 and 7 ; an aqueous solution was then added with 156 mg of isoleucine ; the reaction was regulated with a pH of between 7 and 8 with an aqueo-ammoniacal solution for 5 minutes ; it was immediately passed through a millipore filter ; it was concentrated by slight heating and distilled in vacuum until the volume had reduced by half ; when the mixture cooled, the aromatic complex based on copper isoleucinate was precipitated ; it was filtered and recrystalized in ethanol/water (ratio : 30/70).

The crystals obtained were royal blue in colour and their characterization was the following :

COMPOUND FORMULA : [Cu (1, 10-phenanthroline) (isoleucinate)]$^+$ NO$_3$

ELEMENT ANALYSIS OBTAINED (%) : 49.4 C ; 4.7 H ; 12.9 N

CALCULATED ELEMENT ANALYSIS (%) : (49.60) C ; (4.62) H ; (12.85) N

BORH MAGNETONS ($\mu$, eff) : 1.83

CONDUCTIVITY (Ohms. mol. cm$^{-1}$) : 28.5 (ethanol)

max. visible (nm) : 610

IR (cm$^{-1}$) : 1650, 1580, 1525, 1380, 1110, 875, 854, 825, 780, 650, 580, 430.

## EXAMPLE 6

A complex with the formula [Cu (1, 10-phenanthroline) (cysteinate)]$^+$ NO$_3$ was obtained, the intermediary complex was prepared with the formula [Cu (1, 10-phenanthroline)]$^+$ NO$_3$ following the technique described in examples 1 and 2 and its pH was regulated at between 5 and 7 ; an aqueous solution with 145.9 mg of cystein was then added and the reaction regulated with a pH of between 7 and 8 with an aqueo-ammoniacal solution for 5 minutes ; the product obtained was immediately passed through a millipore filter ; it was concentrated by means of slight heating and distilled in vacuum until the volume had been reduced by half ; when the mixture cooled, the aromatic complex based on copper cysteinate was precipitated ; it was filtered and recrystalized in ethanol/water (ratio : 30/70).

The crystals obtained were royal blue in colour and their characterization was the following :

COMPOUND FORMULA : [Cu (1, 10-phenanthroline) (cysteinate)]$^+$ NO$_3$

ELEMENT ANALYSIS OBTAINED (%) : 42.35 C; 3.34 H ; 13.14 N

CALCULATED ELEMENT ANALYSIS (%) : (42.3) C ; (3.31) H ; (13.15) N

BORH MAGNETONS ($\mu$, eff) : 1.82

CONDUCTIVITY (Ohms. mol. cm$^{-1}$) : 61 (nitrobenzene)

max : visible (nm) : 423

IR (cm $^{-1}$) : 1645, 1380, 1135, 1105, 1005, 835, 757, 669, 642, 570.

## EXAMPLE 7

A complex with the formula [Cu (1, 10-phenanthroline) (diglycinate)]$^+$ NO$_3$ was obtained ; the intermediary complex was prepared with the formula [Cu (1, 10-phenanthroline)]$^+$ NO$_3$, following the technique described in examples 1 and 2 ; its pH was regulated at between 5 and 7 ; an aqueous solution with 158.4 mg of diglycine was subsequently added and the reaction regulated at pH between 7 and 8 with an aqueo-ammoniacal solution for 5 minutes ; the product obtained was immediately passed through a millipore filter ; it was concentrated by means of slight heating and distilled in vacuum until half the volume remained ; when the mixture cooled the aromatic complex based on copper diglycinate was precipitated ; it was filtered and recrystalized in ethanol/water (ratio : 30/70).

The crystals obtained were royal blue in colour and their characterization was the following :

COMPOUND FORMULA : [Cu (1, 10-phenanthroline) (diglycinate)]$^+$ $NO_3$

ELEMENT ANALYSIS OBTAINED : (%) : 43.5 C ; 3.6 H ; 12.92 N

CALCULATED ELEMENT ANALYSIS (%) : (43.92) C ; (3.43) H ; (12.82) N

BORH MAGNETONS ($\mu$, eff) : 1.79

CONDUCTIVITY (Ohms. mol. cm$^{-1}$) : 100 (water)

max : visible (nm) : 615

IR (cm $^{-1}$) : 1628, 1520, 1380, 1172, 1107, 852, 723, 650, 430.

## EXAMPLE 8

A complex with the formula [Cu (1, 10-phenanthroline) (phenylalaninate)]$^+$ $NO_3$ was obtained ; the intermediary complex was prepared with the formula [Cu (1, 10-phenanthroline)]$^+$ $NO_3$, following the technique described in examples 1 and 2, regulating its pH at between 5 and 7, an aqueous solution with 104.4 mg of phenylalanine was subsequently added, the reaction was regulated at a pH of between 7 and 8 with an aqueo-ammoniacal solution for 5 minutes ; the product obtained was immediately passed through a millipore filter ; it was concentrated by means of slight heating and distilled in vacuum until half the volume remained ; when the mixture cooled, the aromatic complex based on copper phenylalaninate was precipitated ; it was filtered and recrystalized in ethanol/water (ratio 30/70).

The crystals obtained were royal blue in colour and their characterization was the following :

COMPOUND FORMULA : [Cu (1, 10-phenanthroline) (phenylalaninate)]$^+$ $NO_3$

ELEMENT ANALYSIS OBTAINED (%) : 51.43 C ; 4.44 H ; 11.34 N

CALCULATED ELEMENT ANALYSIS (%) : (51.69) C ; (4.10) H ; (11.48) N

BORH MAGNETONS ($\mu$, eff) : 1.76

CONDUCTIVITY (Ohms. mol. cm$^{-1}$) : 123 (water)

max visible (nm) : 610

IR (cm $^{-1}$) : 1585, 1520, 1380, 1108, 855, 830, 769, 740, 670, 650, 639, 555, 440.

## EXAMPLE 9

A complex with the formula [Cu (1, 10-phenanthroline) (glycinate)]$^+$ $NO_3$ was obtained ; the intermediary complex was prepared with the formula [Cu (1, 10-phenanthroline)]$^+$ $NO_3$, following the technique described in examples 1 and 2 and its pH was regulated at between 5 and 7 ; an aqueous solution with 89 mg of glycine was subsequently added and the reaction regulated at a pH of between 7 and 8 with an aqueo-ammoniacal solution for 5 minutes ; the product obtained was immediately passed through a millipore filter ; it was concentrated by means of slight heating and distilled in vacuum until half the volume remained ; when the mixture cooled, the aromatic complex based on copper glycinate was precipitated; it was filtered and recrystalized in ethanol/water (ratio : 30/70).

The crystals obtained were royal blue in colour and their characterization was the following :

COMPOUND FORMULA : [Cu (1, 10-phenanthroline) (glycinate)]$^+$ $NO_3$

ELEMENT ANALYSIS OBTAINED (%) : 44.25 C ; 3.12 H ; 14.26 N

CALCULATED ELEMENT ANALYSIS (%) : (44.27) C ; (3.16) H ; (14.75) N

BORH MAGNETONS ($\mu$, eff) : 1.85

CONDUCTIVITY (Ohms. mol. cm$^{-1}$) : 126 (water)

max : visible (nm) : 615

IR (cm $^{-1}$) : 1580, 1520, 1380, 1225, and 1105, 1050, 855, 780, 725, 665, 650, 440.

## EXAMPLE 10

A complex with the formula [Cu (1, 10-phenanthroline) (hystidinate)]$^+$ $NO_3$ was obtained ; the intermediary complex was prepared with the formula [Cu (1, 10-phenanthroline)]$^+$ $NO_3$, following the technique described in examples 1 and 2 regulating its pH at between 5 and 7 ; an aqueous solution with 176.4 mg of hystidine was subsequently added and the reaction regulated at a pH of between 7 and 8 with an aqueo-ammoniacal solution for 5 minutes ; the product obtained was immediately passed through a millipore filter ; it was concentrated by slight heating and distilled in vacuum until half the volume remained ; when the mixture cooled, the aromatic complex based on copper hystidinate was precipitated ; it was filtered and recrystalized in ethanol/water (ratio : 30/70).

The crystals obtained were royal blue in colour and their characterization was the following.

COMPOUND FORMULA : [Cu (1, 10-phenanthroline) (hystidinate)]$^+$ $NO_3$

ELEMENT ANALYSIS OBTAINED (%) : 49.0 C ; 3.56 H ; 15.65 N

CALCULATED ELEMENT ANALYSIS (%) : (48.8) C ; (13.59) H ; (15.65) N

BORH MAGNETONS ($\mu$, eff) : 1.65

CONDUCTIVITY (Ohms. mol. cm$^{-1}$) : 214 (water)

max : visible (nm) : 665

IR (cm $^{-1}$) : 1590, 1525, 1380, 1149, 1110, 855, 838, 780, 725, 675, 653, 565, 440

## EXAMPLE 11

A complex with the formula [Cu (1, 10-phenanthroline) (serinate)]$^+$ $NO_3$ was obtained ; the intermediary complex with the formula [Cu (1, 10-phenanthroline)]⁺ NO₃ was prepared, following the technique described in examples 1 and 2, regulating its pH at between 5 and 7 ; an aqueous solution with 124.8 mg of serine was subsequently added ; the reaction regulated at pH between 7 and 8 with an aqueo-ammoniacal solution for 5 minutes ; the product obtained was immediately passed through a millipore filter ; it was concentrated by slight heating and distilled in vacuum until half the volume remained ; when the mixture cooled, the aromatic complex based on copper serinate was precipitated ; it was filtered and recrystalized in ethanol/water (ratio : 30/70).

The crystals obtained were royal blue in colour and their characterization was the following :

COMPOUND FORMULA : $[Cu (1, 10$-phenanthroline) (serinate)$]^+ NO_3$

ELEMENT ANALYSIS OBTAINED (%) : 44.11 C ; 3.30 H ; 13.68 N

CALCULATED ELEMENT ANALYSIS (%) : (44.07) C ; (3.20) H ; (13.70) N

BORH MAGNETONS ($\mu$, eff) : 1.78

CONDUCTIVITY (Ohms. mol. cm⁻¹) : 90 (water)

max visible (nm) : 612

IR (cm⁻¹) : 1521, 1380, 1170, 1105, 860, 829, 723, 653

## EXAMPLE 12

A complex with the formula $[Cu (1, 10$-phenanthroline) (tyrosinate)$]^+ NO_3$ was obtained the intermediary complex was prepared with the formula $[Cu (1, 10$-phenanthroline)$]^+ NO_3$, following the technique described in examples 1 and 2 regulating pH at between 5 and 7. an aqueous solution with 216.0 mg of tyrosine was subsequently added and the reaction regulated at between pH 7 and 8 with an aqueo-ammoniacal solution for 5 minutes ; the product obtained was immediately passed through a millipore filter ; it was concentrated by slight heating and distillation in vacuum until half the volume remained ; when the mixture cooled, the aromatic complex based on copper tyrosinate was precipitated ; it was filtered and recrystalized in ethanol/water (ratio : 30/70).

The crystals obtained were royal blue in colour and were characterized as follows :

COMPOUND FORMULA : $[Cu (1, 10$-phenanthroline) (tyrosinate)$]^+ NO_3$

ELEMENT ANALYSIS OBTAINED (%) : 49.32 C ; 3.60 H ; 12.35 N

CALCULATED ELEMENT ANALYSIS (%) : (49.40) C ; (3.90) H ; (12.13) N

BORH MAGNETONS ($\mu$, eff) : 1.91

CONDUCTIVITY (Ohms. mol. cm⁻¹) : 136 (water)

max. visible (nm) : 610

IR (cm⁻¹) : 1590, 1520, 1380, 1180, 1110, 915, 880, 850, 815, 780, 745, 650, 550, 440.

## EXAMPLE 13

A complex with the formula $[Cu (1, 10$-phenanthroline) (aspartate)$]^+ NO_3$ was obtained ; the intermediary complex was prepared with the formula $[Cu (1, 10$-phenanthroline)$]^+ NO_3$, following the technique described in examples 1 and 2, regulating pH at between 5 and 7 ; an aqueous solution with 158.4 mg of aspartate was subsequently added and the reaction regulated between pH 7 and 8 with an aqueo-ammoniacal solution for 5 minutes ; the product obtained was immediately passed through a millipore filter ; it was concentrated by slight heating and distillation in vacuum until the volume was reduced by half; when the mixture cooled, the aromatic complex based on copper aspartate was precipitated ; it was filtered and recrystalized in ethanol/water (ratio : 30/70)

The crystals obtained were royal blue in colour and were characterized as follows :

COMPOUND FORMULA : $[Cu (1, 10$-phenanthroline) (aspartate)$]^+ NO_3$

ELEMENT ANALYSIS OBTAINED (%) : 43.8 C ; 3.31 H ; 12.79 N

CALCULATED ELEMENT ANALYSIS (%) : (43.91) C ; (3.22) H ; (12.80) N

BORH MAGNETONS : ($\mu$, eff) : 1.85

CONDUCTIVITY (Ohmos. mol. cm⁻¹) : 104.5 (methanol)

max. visible (nm) : 628

IR (cm⁻¹) : 1609, 1518, 1380, 1143, 1105, 854 and 827, 723, 644.

## EXAMPLE 14

A complex with the formula $[Cu (1, 10$-phenanthroline) (aluminate)$]^+ NO_3$ was obtained, the intermediary complex with the formula $[Cu (1, 10$-phenanthroline)$]^+ NO_3$ was prepared following the technique described in examples 1 and 2 regulating pH between 5 and 7, an aqueous solution with 105.6 mg of alanine was subsequently added and the reaction regulated between pH 7 and 8 with an aqueo-ammoniacal solution for 5 minutes ; the product obtained was immediately passed through a millipore filter ; it was concentrated by slight heating and distillation in vacuum until the volume was reduced by half, when the mixture cooled, the aromatic complex based on copper alaninate was precipitated ; it was filtered and recrystalized in ethanol/water (ratio : 30/70).

The crystals obtained were royal blue in colour and were characterized as follows :

COMPOUND FORMULA : $[Cu (1, 10$-phenanthroline) (alaninate)$]^+ NO_3$

ELEMENT ANALYSIS OBTAINED (%) : 42.96 C ; 3.40 H ; 13.50 N

CALCULATED ELEMENT ANALYSIS (%) : (43.74) C ; (3.88) H ; (13.60) N

BORH MAGNETONS ($\mu$, eff), 1.95

CONDUCTIVITY (Ohms. mol. cm$^{-1}$) : 119 (water)
max. visible (nm) : 609
IR (cm $^{-1}$) : 1590, 1520, 1380, 1150 and 1115, 930, 875, 855, 780, 725, 650, 439.

## EXAMPLE 15

In order to obtain a complex with the formula [Cu (4, 7-dimethyl-1, 10 phenanthroline) (phenylalaninate)]$^+$ NO$_3$, an aqueo-ethanolic solution with 215.6 mg Cu (NO$_3$)$_2$. 5H$_2$O was prepared in a container ; a second aqueo-ethanolic solution with 208 mg of 4, 7-dimethyl-1, 10-phennthroline was immediately prepared ; the aqueous solutions were then made to react in a reactor at room temperature ; soon after a regulation agent was added to stabilize the intermediary complex obtained, keeping the pH at between 5 and 7 ; an aqueous solution with 104.4 mg of phenylalanine was subsequently added to the intermediary complex obtained ; the reaction was kept at a pH between 7 and 8 and was regulated with an aqueo-ammoniacal solution for 5 minutes ; the product obtained was immediately passed through a millipore filter ; it was concentrated by slight heating and distillation in vacuum until the volume was reduced by half ; once the mixture had been cooled, the aromatic complex obtained from copper phenylalaninate was precipitated ; it was then filtered and recrystalyzed in a mixture of ethanol/water (at a ratio of 30/70).

The crystals obtained were royal blue in colour and their characterization was as follows :
COMPOUND FORMULA : [Cu (4, 7-dimethyl-1, 10-phenanthroline) (phenylalaninate)]$^+$ NO$_3$
ELEMENT ANALYSIS OBTAINED (%) : 55.50 C ; 4.30 H ; 11.30 N
CALCULATED ELEMENT ANALYSIS (%) : (55.47) C ; (4.21) H ; (11.25) N
BORH MAGNETONS ($\mu$, eff) : 2.07
CONDUCTIVITY (Ohms. mol. cm$^{-1}$) : 125 (water)
max. visible (nm) : 609
IR (cm $^{-1}$) : 1580, 1525, 1380, 1230, 1170, 1025, 865 and 855, 749 and 720, 700, 665, 645, 570, 430

## EXAMPLE 16

To obtain a complex with the formula [Cu (4, 7 dimethyl-1, 10-phenanthroline) (glycinate)]$^+$ NO$_3$, an aquo-ethanolic solution with 215.6 mg of Cu (NO$_3$)$_2$·5H$_2$O was prepared in a vessel ; a second aqueo-ethanolic solution with 208 mg of 4, 7 dimethyl-1, 10-phenanthroline was immediately prepared ; the aqueous solutions were then made to react in a reactor at room temperature ; soon after a regulation agent was added to stabilize the intermediary complex obtained, keeping the pH at between 5 and 7, an aqueous solution with 89 mg of glycinate was subsequently added to the intermediary complex obtained ; the reaction was kept at a pH of beetween 7 and 8 and was regulated with an aqueo-ammoniacal solution for 5 minutes ; the product obtained was immediately passed through a millipore filter ; it was concentrated by slight heating and distillation in vacuum until the volume was reduced by half ; once the mixture had cooled, the intermediary complex obtained from copper glycinate was precipated ; it was then filtered and recrystalized in an ethanol/water mixture (at a ratio of 30/70).

The crystals obtained were royal blue in colour and their characterization was as follows :
COMPOUND FORMULA : [Cu (4, 7-dimethyl-1, 10-phenanthroline) (glycinate)]$^+$ NO$_3$
ELEMENT ANALYSIS OBTAINED (%) : 47.20 C ; 4.0 H ; 13.80 N
CALCULATED ELEMENT ANALYSIS (%) : (47.12) C ; (3.93) H ; (13.74) N
BORH MAGNETONS ($\mu$, eff) : 1.72
CONDUCTIVITY (Ohms. mol. cm$^{-1}$) : 112 (water)
max. visible (nm) : 603.7
STRUCTURE (R) : 0.046
IR (cm $^{-1}$) : 1579, 1525, 1380, 1170, 1120, 1030, 870, 725, 690, 665, 645, 430.

## EXAMPLE 17

To obtain a complex with the formula [Cu (4, 7 dimethyl-1, 10-phenanthroline) (histidinate)]$^+$ NO$_3$, an aqueo-ethanolic solution with 215.6 mg of Cu (NO$_3$)$_2$·5H$_2$O was prepared in a vessel ; a second aqueo-ethanolic solution with 208 mg of 4, 7-dimethyl-1, 10-phenanthroline was immediately prepared; the aqueous solutions were then made to react in a reactor at room temperature ; soon after a regulating agent was added to stabilize the intermediary complex obtained, keeping the pH at between 5 and 7 ; and aqueous solution with 176.4 mg of histidine was subsequently added to the intermediary complex obtained, the reaction was kept at a pH of between 7 and 8 and was regulated with an aqueo-ammoniacal solution for 5 minutes ; the product obtained was immediately passed through a millipore filter ; it was concentrated by slight heating and distillation in vacuum until the volume had been reduced by half ; once the mixture had cooled, the aromatic complex obtained from copper histidinate was precipitated ; it was then filtered and recrystalized in an ethanol/water mixture (with a ratio of 30/70).

The crystals obtained were royal blue in colour and were characterized as follows :
COMPOUND FORMULA : [Cu (4, 7-dimethyl-1, 10 phenanthroline) (histidinate)]$^+$ NO$_3$
ELEMENT ANALYSIS OBTAINED (%) : 49.10 C ; 4.2 H ; 17.3 N
CALCULATED ELEMENT ANALYSIS (%) : (49.22) C ; (4.10) H ; (17.23) N
BORH MAGNETONS ($\mu$, eff) : 1.62
CONDUCTIVITY (Ohms. mol. cm$^{-1}$) : 205 (water)

max. visible (nm) : 666.5

IR (cm $^{-1}$) : 1640, 1485, 1455, 1380, 1255, 1160, 1105, 1039, 840, 780, 655, 560, 430.

## EXAMPLE 18

In order to obtain a complex with the formula [Cu (4, 7-dimethyl-1, 10-phenanthroline) (tyrosinate)]$^+$ NO$_3$, an aqueo-ethanolic solution with 215.6 mg of Cu (NO$_3$)$_2$·5H$_2$O was prepared in a vessel ; a second aqueo-ethanolic solution with 208 mg of 4, 7-dimethyl-1, 10-phenanthroline was immediately prepared ; the aqueous solutions were then made to react in a reactor at room temperature ; soon after a regulating agent was added to stabilize the intermediary product obtained, keeping the pH at between 5 and 7 ; an aqueous solution with 216 mg of tyrosine was then added to the intermediary complex obtained, the reaction was kept at a pH of between 7 and 8 and was regulated with an aqueo-ammoniacal solution for 5 minutes ; the product obtained was immediately passed through a millipore filter ; it was concentrated by slight heating and distillation in vacuum until the volume had been reduced by half ; once the mixture had cooled, the aromatic complex obtained from copper tyrosinate was precipitated ; it was then filtered and recrystalized in an ethanol/water mixture (at a ratio of 30/70).

The crystals obtained were royal blue in colour and were characterized as a follows :

COMPOUND FORMULA : [Cu (4, 7-dimethyl-1, 10-phenanthroline) (tyrosinate)]$^+$ NO$_3$

ELEMENT ANALYSIS OBTAINED (%) : 53.81 C ; 4.31 H ; 10.89 N

CALCULATED ELEMENT ANALYSIS (%) : (53.74) C ; (4.28) H ; (10.90) N

BORH MAGNETONS (μ, eff) : 1.96

CONDUCTIVITY (Ohms. mol. cm$^{-1}$) : 125 (water)

max. visible (nm) : 613.9

IR (cm $^{-1}$) : 1615, 1580, 1525, 1380, 1175, 1120, 980, 935, 870, 850 and 815, 725, 690, 650, 540.

## EXAMPLE 19

In order to obtain a complex with the formula [Cu (4, 7-dimethyl-1, 10-phenanthroline) (alaninate)]$^+$ NO$_3$, an aqueo-ethanolic solution with 215.6 mg of Cu (NO$_3$)$_2$·5H$_2$O was prepared in a vessel ; a second aqueo-ethanolic solution with 208 mg of 4, 7-dimethyl (1, 10-phenanthroline) was immediately prepared ; the aqueous solutions were then made to react in a reactor at room temperature : soon after a regulating agent was added to stabilize the intermediary complex obtained, keeping the pH at between 5 and 7 ; an aqueous solution with 105.6 mg of alanine was subsequently added to the intermediary complex obtained ; the reaction was kept at a pH of between 7 and 8, and was regulated with an aqueo-ammoniacal solution for 5 minutes ; the product obtained was immediately passed through a millipore filter ; it was concentrated by slight heating and distillation in vacuum until the volume had been reduced by half ; once the mixture had cooled, the aromatic complex based on copper alaninate was precipitated ; it was then filtered and recrystalized in an ethanol/water mixture (at a ratio of 30/70).

The crystals obtained were royal blue in colour and were characterized as follows :

COMPOUND FORMULA : [Cu (4, 7-dimethyl-1, 10-phenanthroline) (alaninate)]$^+$ NO$_3$

ELEMENT ANALYSIS OBTAINED (%) : 55.5 C ; 4.3 H ; 11.3 N

CALCULATED ELEMENT ANALYSIS (%) : (55.47) C ; (4.21) H ; (11.25) N

BORH MAGNETONS (μ, eff) : 1.89

CONDUCTIVITY (Ohms. mol. cm$^{-1}$) : 117 (water)

max. visible (nm) : 601.5

IR (cm $^{-1}$) : 1610, 1555, 1380, 1161, 1110, 1065, 920, 850, 740 and 730, 695, 605, 430.

## Claims

1. The process to obtain new mixed copper aminoacidate complexes from phenanthrolines and their alkyl derivates to be used as anticancerigenic agents of an aromatic type with the formula [Cu (N-N) (N-O)]$^+$ NO$_3$, where the N-N ligand (diimine) corresponds to a group of 1, 10 phenanthroline and their alkyl derivates, preferably 4, 7-dimethyl-1, 10-phenanthroline, and the N-O ligand is an aminoacidate corresponding principally to the following ions : tyrosin-alaninate, treoninate, triptophanate, valinate, isoleucinate, cysteinate, diglycinate, phenylalaninate, glycinate, histidinate, serinate, tirosinate, aspartate, alaninate and phenylalaninate ; the process is characterized because it comprises the steps to be taken in preparing an aqueous solution based on a copper compound, preferably Cu (NO$_3$)$_2$·5H$_2$O with a ratio of 30-35% in weight of copper/solution ; to prepare a second aqueo-ethanolic solution with a diimine compound with a ratio of 50-55% in weight of diimine/solution ; to make react the aqueous solutions prepared in a reactor at room temperature ; to stabilize the copper complex obtained (nitrate monodiimine) with a regulating agent, keeping an acid pH of between 5 and 7 ; to add and aqueous solution 10-15% in weight of an aminoacidate (N-O) to the complex obtained, keeping the reaction slightly alkaline to obtain aromatic copper aminoacidate complexes, with anticancerigenic properties, from any of the following :

   [Cu (1, 10-phenanthroline) (tirosin-alaninate)]$^+$ NO$_3$ ;

[Cu (1, 10-phenanthroline) (treoninate)]$^+$ NO$_3$;

[Cu (1, 10-phenanthroline) (thriptophanate)]$^+$ NO$_3$;

[Cu (1, 10-phenanthroline) (valinate)]$^+$ NO$_3$;

[Cu (1, 10-phenanthroline) (isoleucinate)]$^+$ NO$_3$;

[Cu (1, 10-phenanthroline) (cysteinate)]$^+$ NO$_3$;

[Cu (1, 10-phenanthroline) (diglycinate)]$^+$ NO$_3$;

[Cu (1, 10-phenanthroline) (phenylalaninate)]$^+$ NO$_3$;

[Cu (1, 10-phenanthroline) (glycinate)]$^+$ NO$_3$;

[Cu (1, 10-phenanthroline) (hystidinate)]$^+$ NO$_3$;

[Cu (1, 10-phenanthroline) (serinate)]$^+$ NO$_3$;

[Cu (1, 10-phenanthroline) (tyrosinate)]$^+$ NO$_3$;

[Cu (1, 10-phenanthroline) (aspartate)]$^+$ NO$_3$;

[Cu (1, 10-phenanthroline) (alaninate)]$^+$ NO$_3$;

[Cu (4, 7-dimethyl-1, 10-phenanthroline) (phenylalaninate)]$^+$ NO$_3$;

[Cu (4, 7 dimethyl-1, 10-phenanthroline) (glycinate)]$^+$ NO$_3$;

[Cu (4, 7 dimethyl-1, 10-phenanthroline) (histidinate)]$^+$ NO$_3$;

[Cu (4, 7 dimethyl-1, 10-phenanthroline) (tyrosinate)]$^+$ NO$_3$ and

[Cu (4, 7-dimethyl-1, 10-phenanthroline) (alaninate)]$^+$ NO$_3$;

2. The process to obtain the new mixed copper aminoacidate complexes from phenanthrolines and their alkyl derivates to be used as anticancerigenic agents, in accordance with claim 1, characterized because a hydrated nitrate salt with the formula Cu (NO$_3$)$_2$·5H$_2$O is used as complex copper reagent with which it is possible to obtain highly water soluble crystals, and because the reaction takes place at room temperature, the aminoacidate obtained conserves its therapeutic properties.

3. A process to obtain new mixed copper aminoacidate complexes from phenanthrolines and their alkyl derivates to be used as anticancerigenic agents in accordance with claims 1 and 2, characterized because the intermediary copper complex of the [Cu (N-N)]$^+$ NO$_3$ type is formed, which is prepared in an aqueo-ethanolic solution with a content of 30-35% in weight of Cu (NO$_3$)$_2$·5H$_2$O and an aqueo-ethanolic solution of 50-55% in weight of the 1, 10-phenanthroline ; these solutions are immediately brought into contact in a reactor for approximately 5 minutes at room temperature, the resulting pH must be between 5 and 7 to obtain the intermediary complex.

4. A process to obtain new mixed copper aminoacidate complexes from phenanthrolines and their alkyl derivates to be used as anticancerigenic agents in accordance with claims 1 to 3, characterized because the intermediary copper complex of the [Cu (N-N)]$^+$ NO$_3$ type is formed, which is prepared in an aqueo-ethanolic solution with a content of 30-35% in weight of Cu (NO$_3$)$_2$·5H$_2$O and an aqueo-ethanolic solution of 50-55% in weight of the 4, 7-dimethyl-1, 10-phenanthroline; these solutions are immediately brought into contact in a reactor for approximately 5 minutes at room temperature, the resulting pH must be between 5 and 7 to obtain the intermediary complex.

5. A process to obtain new mixed copper aminoacidate complexes from phenanthrolines and their alkyl derivates to be used as anticancerigenic agents in accordance with claims 1 to 4, characterised because in order to form the stabilized copper complex of the [Cu (N-N) (N-O)]$^+$ NO$_3$ type, an aqueous solution is prepared, preferably with 12 to 15% in weight of the aminoacidate ion (tyrosin-alaninate, treoninate, triptophanate, valinate, isoleucinate, cysteinate, diglycinate, phenylalaninate, glycinate, histidinate, serinate, tyrosinate, aspartate, alaninate or phenylalaninate) and is made to react with the intermediary copper complex of the [Cu (N-N)]$^+$ NO$_3$ type, adjusting the slightly alkaline pH with an aqueo-ammoniacal solution.

6. A process to obtain new mixed copper aminoacidate complexes from phenanthrolines and their alkyl derivates to be used as anticancerigenic agents in accordance with claims 1 to 5, charaterized because the new compounds of the general formula : [Cu (N-N) (N-O)]$^+$ NO$_3$ are useful for the treatment of liquid and solid cancerigenic tumours such as leukemia.